# NEUE EUROPÄISCHE PATENTSCHRIFT

(11) **EP 1 270 023 B2**
(45) Veröffentlichungstag und Bekanntmachung des Hinweises auf die Entscheidung über den Einspruch: **11.04.2012**
(45) Hinweis auf die Patenterteilung: 15.06.2005
(21) Anmeldenummer: 02019905.5
(22) Anmeldetag: 17.07.1998
(51) Int. Cl.: A61L 31/00

(54) **In vivo abbaubare metallische Gefäßstütze**
Metallic stent which is degradable in vivo
Stent métallique dégradable in vivo

(30) Priorität: 18.07.1997 DE 19731021
(43) Veröffentlichungstag der Anmeldung: 02.01.2003
(62) Teilanmeldung aus: 98943732.2
(73) Patentinhaber: Fischer, Alfons, 45239 Essen (DE); Hausdorf, Jacqueline Yvonne, 30938 Burgwedel (DE); Heublein, Eva, 30627 Hannover (DE)
(72) Erfinder: Heublein, Bernd Prof. Dr., VERSTORBEN (DE); Hausdorf, Gerd Prof. Dr., VERSTORBEN (DE)
(74) Vertreter: Gesthuysen, von Rohr & Eggert

(56) Entgegenhaltungen:
- GB-A- 319 886
- US-A- 3 687 135
- US-A- 5 306 286
- P.N. Sawyer et al., Vol. XIII Trans. Amer. Soc. Artif. Int. Organs, 1967, pp. 124-130

## Beschreibung

Die vorliegende Erfindung betrifft eine Gefäßstütze aus metallischen Werkstoffen zum Einsatz in dem menschlichen oder tierischen Körper.

Implantate sind grundsätzlich seit langem bekannt. Die ersten Implantate wurden zu orthopädischen Zwecken entwickelt, beispielsweise Schrauben und Nägel zum Fixieren von Knochenbrüchen. Diese bestanden zunächst aus relativ einfachen Eisenlegierungen, die unter in vivo-Bedingungen zu Korrosion neigten. Die Korrosion führte dazu, dass in unmittelbarer Nähe des Knochens Metalle als Ionen freigesetzt wurden, die einen unerwünschten Anreiz für das Wachstum des Knochengewebes gegeben haben. Der Knochen ist stärker gewachsen, als es eigentlich erwünscht und erforderlich ist. Hierdurch wurde das gesunde Knochenmaterial geschädigt.

Aus diesem Grund ist man bestrebt, metallische Implantate grundsätzlich aus möglichst korrosionsbeständigen Materialien zu fertigen. Hier sind derzeit hauptsächlich korrosionsbeständige Edelstähle, Tantal und Titan im Gebrauch. Diese Implantate bleiben nach der Implantierung als Fremdkörper präsent und werden als solche vom Organismus erkannt. Sie sind nur durch eine zweite Operation zu entfernen.

Außerdem sind metallische Implantate im Bereich der Gefäßchirurgie und der Kardiologie, Angiologie und Radiologie bekannt. Diese Implantate umfassen zum Beispiel endoluminale und Gefäßstützen (Stents) zur Behandlung von Läsionen. Diese Stützen dienen z. B. zur Aufweitung und Lumenerhaltung von verengten Gefäßen, indem sie vom Gefäßlumen ausgehend mit einem Ballonkatheter (balloon expandable) oder selbstexpandierend (self expanding) das Gefäßlumen auf einem entsprechend optimalen Innendurchmesser halten. Das Implantat ist an sich nur so lange erforderlich, bis das erkrankte Gefäß durch biologische Reparaturvorgänge aus eigener Kraft den erforderlichen Durchmesser dauerhaft halten kann. Dies ist im allgemeinen etwa 4 Wochen nach Implantation der Fall.

Der dauerhafte Verbleib eines metallischen Implantats ist jedoch mit einigen Nachteilen verbunden. Das Implantat führt als Fremdkörper zu lokalen und eventuell auch systemischen Reaktionen. Zusätzlich wird die Selbstregulation des betroffenen Gefäßsegments behindert. Die ständige (pulsatile) Belastung des Metalls kann zu Ermüdungsbrüchen führen, was bei großlumigen Implantaten (z. B. Verschlusssystemen wie Schirmchen) zu neuen medizinischen Problemen führen kann. Gefäßstützen in kleineren Lumina (2,5 - 6 mm) erzeugen in etwa 20% eine erneute Stenosierung (sogenannte In-Stent-Stenose), was bei der hohen Zahl der Implantate kumulativ zu einer zusätzlichen medizinischen und ökonomischen Belastung führt. In einigen Gefäßregionen (z. B. extrakranielle Gefäße, Beinarterien) kann die metallische Struktur durch Krafteinwirkung von außen dauerhaft verformt werden mit den Folgen einer erneuten Gefäßobstruktion bzw. eines induzierten Gefäßverschlusses. Jedes Dauerimplantat ist zusätzlich mit Problemen insbesondere für jüngere Patienten deshalb verbunden, weil ein Verbleiben für Jahrzehnte unausweichlich ist.

Vollkommen biologisch abbaubare Implantate sind bislang nur aus Kunststoffmaterialien bekannt, beispielsweise aus der DE 2502884 C2. Dort wird eine Beschichtung eines orthopädischen Implantats mit Polymethylmethacrylat offenbart, das biodegradabel ist. Andere Kunststoffmaterialien umfassen Polylactidund Polyglycolsäureester. Außerdem ist aus der EP 0006544 B1 ein biodegradables Keramikmaterial auf Basis von Calciumphosphat bekannt, das ebenfalls zur Beschichtung von metallischen Implantaten dient.

Schließlich ist aus der WO 81/02668 ein orthopädisches Implantat bekannt, das einen korrosionsbeständigen metallischen Grundkörper sowie eine biologisch abbaubare, metallische Zwischenschicht für den Kontaktbereich zum Knochen ausweist. Diese Zwischenschicht bildet zusammen mit dem Grundkörper eine elektrochemische Zelle und erzeugt eine elektrische Spannung, die das Knochenwachstum fördert. Gleichzeitig wird die Oberflächenschicht, die beispielsweise aus Silberlegierungen bestehen kann, abgebaut. Dies führt zu dem angestrebten Effekt, dass das Knochenwachstum so lange positiv beeinflusst wird, wie es erforderlich ist und dann nach vollständigem Abbau der Oberflächenbeschichtung der elektrische Reiz nachlässt.

Bisher bekannte biodegradable Substanzen auf Polymerbasis werden in der Gefäßchirurgie verwendet. Ihre mechanischen Eigenschaften einerseits und die nachfolgende Fremdkörperreaktion während der Biodegradation andererseits führen dazu, dass sie als alleiniges Material für eine Implantation ungeeignet sind. Metallische Werkstoffe/Legierungen besitzen günstige mechanische Eigenschaften (Elastizität, Verformbarkeit, Stabilität) bei geringerer Masse, was für die Applikation durch dünnlumige Führungssysteme bei transkutanem Vorgehen eine wichtige Voraussetzung darstellt.

Es ist daher Aufgabe der vorliegenden Erfindung, eine Gefäßstütze aus biodegradablem Material zur Verfügung zu stellen, die zugleich vorteilhafte mechanische Eigenschaften aufweist.

Diese Aufgabe wird durch Gefäßstützer mit den Merkmalen des Anspruchs 1 gelöst. Weitere vorteilhafte Ausgestaltungen sind Gegenstand der Unteransprüche.

Weil das medizinische Implantat aus einem metallischen Werkstoff gefertigt ist, der durch Korrosion in vivo abbaubar ist, liegen primär die mechanischen Vorteile metallischer Werkstoffe vor. Der korrosive Abbau des Implantats innerhalb einer durch Materialwahl einstellbaren Zeitskala verhindert . andererseits, dass die negativen Langzeiteffekte des metallischen Fremdkörpers eintreten. Dabei ist es biologisch vorteilhaft, wenn der Werkstoff eine Legierung ist deren Hauptbestandteil Magnesium ist, wie sie in Anspruch 1 definiert ist.

Vorteilhafte Abbauzeiten haben sich somit ergeben bei Werkstoffen mit der anspruchsgemässen Zusammensetzung.

Das medizinische Implantat wird als Gefäßstütze ausgeführt. Für eine Gefäßstütze ist als Grundkörper ein rohrförmiger Aufbau mit zusätzlicher Bearbeitung vorgesehen.

Zur Einstellung der Korrosionsgeschwindigkeit des Werkstoffs ist von Vorteil, wenn die Materialstärke des Werkstoffs in Abhängigkeit von der Zusammensetzung des Werkstoffs so gewählt ist, dass der Abbau- oder Korrosionsvorgang in vivo zwischen 5 Tagen und 6 Monaten, insbesondere zwischen 2 Wochen und 8 Wochen, im wesentlichen abgeschlossen ist.

Hierbei wird erreicht, dass nach einem Anwachsen des Gewebeimplantats die dann nicht mehr benötigte Fixiervorrichtung verschwindet.

Im folgenden werden verschiedene Ausführungsbeispiele gegeben.

### Beispiel 1: Gefäßstütze (Bezugsbeispiel)

Ein Stent wird aus einem rohrförmigen Grundkörper des metallischen und nachfolgender Bearbeitung gefertigt. Vom mechanischen Aufbau her sind derartige Stents beispielsweise aus der EP 0221570 B1 bekannt, wobei das Material jedoch ein korrosionsbeständiger Edelstahl ist.

Bei dem Stent nach diesem Beispiel ist das Material eine Legierung mit dem Hauptbestandteil Magnesium und gegebenenfalls den Nebenbestandteilen Lithium, Eisen, Zink und Spuren von Nickel. Die prozentuale Zusammensetzung der Magnesiumlegierung soll etwa im Bereich von 50 - 98% Magnesium, 0 - 40% Lithium, 0 - 5% Eisen und unter 5% andere Metalle liegen. Die Wandstärke der Stentstreben soll nach der Bearbeitung zwischen 50 und 100 µm betragen.

In der Praxis wird der Stent in an sich bekannter Weise mit einem Ballonkatheter in ein krankhaft verengtes Blutgefäß eingesetzt und dort dilatiert oder als selbstexpandierender Stent freigesetzt, wobei er das Blutgefäß auf dem gewünschten Durchmesser hält. Eine ohne Stent-Implantation verbleibende Restenose (Recoil) und/oder ein durch die Dilatation induzierter Geweberiss werden wirkungsvoll behandelt. Innerhalb von 2 - 4 Wochen wird der Stent von Intimagewebe überdeckt und behält seine Stützfunktion zunächst bei. Das Blutgefäß erhält durch Gewebewachstum infolge von Eigenreparaturvorgängen im Bereich des implantierten Stents eine neue Eigenstabilität. Das Gefäßlumen wird auf einem optimalen Niveau stabilisiert. Die Wahl des Legierungsmaterials zusammen mit der gewählten Wandstärke führen andererseits dazu, dass der Stent in der Wandung des Blutgefäßes allmählich abgebaut wird und nach etwa 4 - 12 Wochen nur noch in Spuren vorliegt. Die auf Seite 2 geschilderten Nachteile eines Dauerimplantats gehen verloren.

### Beispiel 2: Verschlusssystem (nicht erfindungsgemäß)

Ein Verschlusssystem (Schirmchen) wird aus einem metallischen Skelett, an dem ein Kunststoffschirmchen befestigt ist, gefertigt. Derartige Schirmchen sind bekannt beispielsweise aus der Legierung MP35N oder Nitinol. Derartige Verschlusssysteme werden zum Verschluss von Defekten in den Herzscheidewänden verwendet. Die Wandstärke des metallischen Gerüstes beträgt um 500 mm. In der Praxis wird das Schirmchen in an sich bekannter Weise zusammengefaltet und in dem zu verschließenden Defekt freigesetzt. Innerhalb von 3 - 4 Wochen wird das Schirmchen vom körpereigenen Gewebe bedeckt und erhält durch dieses Gewebewachstum eine neue Eigenstabilität. Die Wahl des Legierungsmaterials zusammen mit der Gewebewandstärke führt dazu, dass das metallische Gerüst innerhalb von 4 Wochen bis einigen Monaten abgebaut wird und nach einem Jahr nur noch in Spuren vorliegt. Der Kunststoffanteil des Schirmchens bleibt erhalten, was aufgrund der Flexibilität des Materials unkritisch ist. Der Abbau des metallischen Anteils hat gegenüber den bekannten Schirmchen den Vorteil, dass auch bei unvorhergesehenen Belastungen z.B. bei Verkehrsunfällen keine Gefahr des Durchstoßens von Gefäßwandungen mehr besteht. Dabei wird der erfindungsgemäße Vorteil bereits erreicht, wenn zunächst durch die Degradation eine mechanische Instabilität des Gerüsts entsteht.

### Beispiel 3: Spirale zum Verschließen von Gefäßen (Okkluder) (nicht erfindungsgemäß)

Eine erfindungsgemäße Spirale (Coil) wird aus einem in Helixform gewickelten metallischen Material gefertigt und die Spirale vorgebogen. Der Durchmesser der Primärwicklung beträgt 0,1 - 1 mm, je nach dem zu verschließenden Gefäß. Derartige Spiralen (Coils) sind bekannt, beispielsweise aus Nitinol, Platinlegierungen oder Wolframlegierungen.

In der Praxis wird die Verschlussspirale (Coil) in an sich bekannter Weise in gestrecktem Zustand in einen Herzkatheter eingeführt und durch diesen bis zu dem zu verschließenden Gefäß vorgeschoben. Bei der Freisetzung aus dem Herzkatheter nimmt die Spirale wieder ihre alte Form an und verschließt durch ihr Lumen und ihre Thrombogenität, die durch Dacron oder andere Fasern erhöht werden kann, das zu verschließende Gefäß. Nach Thrombosierung des Gefäßes und Einwachsen von Bindegewebe erhält der Verschlussmechanismus eine neue Eigenstabilität. Die applizierte Spirale wird allmählich abgebaut, so dass nach etwa einem Jahr das implantierte Material nur noch in Spuren vorliegt.

Die insoweit genannten Ausführungsbeispiele lassen sich mit Magnesiumlegierungen fertigen. Toxische Wirkungen der Materialien bei den zu erwartenden Konzentrationen sind nicht bekannt.

Magnesiumlegierungen haben den Vorteil, dass durch geeignete Wahl der übrigen Legierungsbestandteile die in vivo zu erwartende Abbaugeschwindigkeit sehr genau gewählt werden kann. Außerdem ist Magnesium physiologisch sehr gut verträglich.

## Patentansprüche

1. Gefäßstütze aus einem durch Korrosion in vivo abbaubaren metallischen Werkstoff,
wobei der Werkstoff eine Legierung ist, deren Hauptbestandteil Magnesium ist, und
wobei der Werkstoff 79 - 97 % Magnesium, 2 - 5 % Aluminium, 0 - 12 % Lithium und 1 - 4 % seltene Erden enthält.

2. Gefäßstütze nach Anspruch 1, **dadurch gekennzeichnet, dass** der Werkstoff als seltene Erden, Cer, Lanthan, Neodym und/oder Praseodym enthält.

3. Gefäßstütze nach Anspruch 1, **dadurch gekennzeichnet, dass** der Werkstoff 85 - 91 % Magnesium, 6 - 12 % Lithium, 2 % Aluminium und 1 % seltene Erden enthält.

4. Gefäßstütze nach Anspruch 1, **dadurch gekennzeichnet, dass** der Werkstoff 86 - 97 % Magnesium, 0 - 8 % Lithium, 2 - 4 % Aluminium und 1 - 2 % seltene Erden enthält.

5. Gefäßstütze nach einem der vorhergehenden Ansprüche, **dadurch gekennzeichnet, dass** die Gefäßstütze einen im wesentlichen rohrförmigen Grundkörper aufweist.

6. Gefäßstütze nach einem der vorhergehenden Ansprüche, **dadurch gekennzeichnet, dass** die Materialstärke des Werkstoffs in Abhängigkeit von der Zusammensetzung des Werkstoffs so gewählt ist, dass der Abbau- oder Korrosionsvorgang in vivo im Bereich von 5 Tagen bis zu 6 Monaten, insbesondere zwischen 2 Wochen und 8 Wochen im wesentlichen abgeschlossen ist.

7. Gefäßstütze nach einem der vorhergehenden Ansprüche 1 bis 5, **dadurch gekennzeichnet, dass** die Materialstärke des Werkstoffs in Abhängigkeit von der Zusammensetzung des Werkstoffs so gewählt ist, dass der Abbau- oder Korrosionsvorgang in vivo im Bereich von 6 Monaten bis zu 10 Jahren, insbesondere zwischen 1 Jahr und 5 Jahren im wesentlichen abgeschlossen ist.

8. Gefäßstütze nach einem der vorhergehenden Ansprüche, **dadurch gekennzeichnet, dass** der Abbau- oder Korrosionsvorgang in vivo zunächst zu einer mechanischen Instabilität führt, bevor der Abbauvorgang im wesentlichen abgeschlossen ist.

## Claims

1. Vascular support made of a metallic material degradable in vivo by corrosion, wherein the material is an alloy, the main constituent of which is magnesium, and
wherein the material contains 79 - 97% of magnesium, 2 - 5% of aluminium, 0 - 12% of lithium and 1 - 4% of rare earths.

2. Vascular support according to Claim 1, **characterised in that** the material contains, as rare earths, cerium, lanthanum, neodymium and/or praseodymium.

3. Vascular support according to Claim 1, **characterised in that** the material contains 85 - 91% of magnesium, 6 - 12% of lithium, 2% of aluminium and 1% of rare earths.

4. Vascular support according to Claim 1, **characterised in that** the material contains 86 - 97% of magnesium, 0 - 8% of lithium, 2 - 4% of aluminium and 1 - 2% of rare earths.

5. Vascular support according to one of the preceding claims, **characterised in that** the vascular support has a substantially tubular base body.

6. Vascular support according to one of the preceding claims, **characterised in that** the material thickness of the material is chosen in dependence on the composition of the material such that the degradation or corrosion process in vivo is substantially completed in the range from 5 days up to 6 months, in particular between 2 weeks and 8 weeks, after implantation.

7. Vascular support according to one of the preceding Claims 1 to 5, **characterised in that** the material thickness of the material is chosen in dependence on the composition of the material such that the degradation or corrosion process in vivo is substantially completed in the range from 6 months up to 10 years, in particular between 1 year and 5 years, after implantation.

8. Vascular support according to one of the preceding claims, **characterised in that** the degradation or corrosion process in vivo results at first in mechanical instability before the degradation process is substantially completed.

## Revendications

1. Endoprothèse vasculaire en matériau métallique dégradable *in vivo* par corrosion, ou le matériau est un alliage dont le constituant principal est le magnésium et
ou le matériau contient 79 à 97% de magnésium, 2 à 5% d'aluminium, 0 à 12% de lithium et 1 à 4% de terres rares.

2. Endoprothèse vasculaire selon la revendication 1, **caractérisée en ce que** le matériau contient, comme les terres rares, le cérium, le lanthane, le néodyme et/ou le praséodyme.

3. Endoprothèse vasculaire selon la revendication 1, **caractérisée en ce que** le matériau contient 85 à 91% de magnésium, 6 à 12% de lithium, 2% d'aluminium et 1 % de terres rares.

4. Endoprothèse vasculaire selon la revendication 1, **caractérisée en ce que** le matériau contient 86 à 97% de magnésium, 0 à 8% de lithium, 2 à 4% d'aluminium et 1 à 2% de terres rares.

5. Endoprothèse vasculaire selon l'une des revendications précédentes, **caractérisée en ce que** l'endoprothèse vasculaire présente un élément de base de forme pratiquement tubulaire.

6. Endoprothèse vasculaire selon l'une des revendications précédentes, **caractérisée en ce que** l'épaisseur du matériau est choisie en fonction de la composition du matériau de telle façon que le processus de dégradation ou de corrosion *in vivo* soit pratiquement achevé après une durée dans la plage de 5 jours à 6 mois, en particulier de 2 à 8 semaines.

7. Endoprothèse vasculaire selon l'une des revendications 1 à 5 précédentes, **caractérisée en ce que** l'épaisseur du matériau est choisie en fonction de la composition du matériau de telle façon que le processus de dégradation ou de corrosion *in vivo* soit pratiquement achevé après une durée dans la plage de 6 mois à 10 ans, en particulier de 1 an à 5 ans.

8. Endoprothèse vasculaire selon l'une des revendications précédentes, **caractérisée en ce que** le processus de dégradation ou de corrosion *in vivo* provoque dans un premier temps une instabilité mécanique avant que le processus de dégradation soit pratiquement achevé.
